# EUROPÄISCHE PATENTANMELDUNG

(11) **EP 1 382 689 A1**
(43) Veröffentlichungstag der Anmeldung: **21.01.2004**
(21) Anmeldenummer: 03014220.2
(22) Anmeldetag: 25.06.2003
(51) Int. Cl.: C12Q 1/34, C12Q 1/527, C12Q 1/24

(54) **Screeningmethode zum Nachweis von Amidase- und Nitrilhydrataseaktivitäten und deren Verwendung**

(30) Priorität: 15.07.2002 AT 10562002
(71) Anmelder: DSM Fine Chemicals Austria Nfg GmbH & Co KG, 4021 Linz (AT)
(72) Erfinder: Reisinger, Christoph, 8042 Graz (AT); Glieder, Anton, 8200 Gleisdorf (AT); Schwab, Helmut, 8043 Graz (AT)
(74) Vertreter: Lindinger, Ingrid

(57) **Zusammenfassung**

Screeningmethode zum Nachweis von Amidase- oder Nitrilhydrataseaktivität, bei welcher
a) ein Replik von Zellkolonien, die Amidasen oder Nitrilhydratasen expremieren mittels eines Trägers hergestellt wird, worauf
   b₁) im Falle von Kolonien mit zu testenden Nitrilhydratasegenen zuerst eine Inkubation der an dem Träger anhaftenden Kolonien mit einer Substratlösung aus einem Nitril der Formel (I) R-CN und einem Puffer und anschließend
   b₂) eine Inkubation mit einer gepufferten Hydroxylammoniumsalzlösung erfolgt, oder
c) im Falle von Zellkolonien mit zu testenden Amidasegenen eine Inkubation der an dem Träger anhaftenden Kolonien mit einer Substratlösung aus einem Amid der Formel (II) R-CONH₂, Hydroxylammoniumsalz und einem Puffer erfolgt, worauf
d) im Anschluss an b₂) oder c) die aktiven Kolonien durch Überführen des Trägers in eine Eisen(III)salzlösung gefärbt werden und
e)gegebenenfalls die aktiven Kolonien isoliert werden,
sowie deren Verwendung.

## Beschreibung

Die Erfindung betrifft eine neue Screeningmethode, die zum Nachweis von Amidaseund Nitrilhydratase-Aktivität auf Ebene von Bakterienkolonien dient, sowie die Verwendung der Methode bei der Klonierung eines Amidase- oder eines Nitrilhydratasegens.

Amidasen sind insbesondere bedeutend für die Hydrolyse von empfindlichen Amiden, bei denen die drastischen Bedingungen der chemischen Hydrolyse nicht anwendbar sind. Man findet sie auch als Folgeenzym von Nitrilhydratasen bei der Hydrolyse von Nitrilen zu Carbonsäuren eingesetzt.
Obwohl bereits mehrere Amidasen, die die unterschiedlichsten Substrate bevorzugen, charakterisiert wurden, wird stets nach Enzymen, die bessere Eigenschaften aufweisen gesucht. Es ist Stand der Technik, für Amidasen kodierende Gene zu klonieren und Amidase-Enzym durch Expression dieser Gene in geeigneten Wirtszellen zu gewinnen.ln den zum Stand der Technik gehörenden Publikationen, beispielsweise in J. Bacteriol. 1991, **173**(21): 6694-704; J. Bacteriol. 1990, **172**(12); 6764-73; Biochim Biophys Acta. 1991, **16**; 1088(2): 225-33 u.s.w., betreffend Klonierung von Amidasen ist der klassische Weg zur Isolation des Gens gewählt worden. Dieser erfolgt durch Ansequenzierung des Peptids, Konstruktion von degenerierten Oligos, Amplifikation einer Sonde mittels PCR und Colony Hybridisierung einer Genbank des Organismus.
In weiterer Folge ist es interessant, Amidasen durch Methoden des Protein Engineering in ihren Eigenschaften zu verbessern, wobei ein wichtiger Ansatz dazu die Strategie des "Directed Evolution" ist. Die Nützlichkeit von "Directed Evolution" und anderen Mutagenese-Strategien ist beispielsweise in Curr Opin Chem Biol.,1999, **3**(1):54-9 und Curr Opin Biotechnol. 2001, **12**(6):545-51 beschrieben.
"Directed Evolution" Programme bei Amidasen benötigen jedoch aufwendige und kostenintensive Analysenmethoden, wie Photometrie, HPLC u.s.w. verwenden, wie beispielsweise in Enzyme and Microbial Technology 2000, **26**: 152-158 beschrieben, da der Nachweis von Carbonsäuren und Ammonium vor einem biologischen Hintergrund keine einfache Aufgabe ist. Das Screenen auf Ammonium aus der Amidhydrolyse als einzige N-Quelle im Medium ist zwar möglich, jedoch sind nur Anreicherungen erzielbar, da Ammonium schnell diffundiert und z.B. *E. coli.* über sehr effektive Aufnahmemechanismen für Ammonium verfügt.
Ebenso wie bei Amidasen, ist auch bei der Klonierung der Nitrilhydratasen die klassische Methode (Proteinreinigung, Sequenzierung, Konstruktion degenerierter Primer, Amplifikation einer Sonde mittels PCR und Colony Hybridisierung einer Genbank oder direktes DNA Screening) als Stand der Technik zu bezeichnen, die beispielsweise in Biosci. Biotechnol. Biochem., 1993 Aug, 57:1323-8; Biochim. Biophys. Acta, 1991 Dez, 1129:23-33 und J. Bacteriol., 1991 Apr, 173:2465-72 u.s.w. beschrieben ist.

Aufgabe der vorliegenden Erfindung war es demnach, eine Methode zu finden, die die Klonierung von Amidasen oder von Nitrilhydratasen ohne Zuhilfenahme von Sequenzinformationen bzw. die Bestimmung von Amidase- oder Nitrilhydratase-Aktivität in Bakterien- bzw. Zellkolonien mit geringem zeitlichen und finanziellen Aufwand ermöglicht und weiters den raschen Vergleich von Amidase- und Nitrilhydratase-Aktivität von großen Zahlen von Organismenkolonien für die Selektion von verbesserten Klonen und Mutanten erlaubt.

Unerwarteterweise konnte diese Aufgabe durch eine 4- bzw. 5-stufige Screeningmethode gelöst werden, die auf der colorimetrischen Anzeige von Amidase-Aktivität beruht.

Gegenstand der vorliegenden Erfindung ist demnach eine Screeningmethode zum Nachweis von Amidase- oder Nitrilhydratase-Aktivität, die dadurch gekennzeichnet ist, dass
a) ein Replika von Zellkolonien, die Amidasen oder Nitrilhydratasen expremieren und die auf einem geeigneten verfestigtem Medium gewachsen sind auf einem geeigneten Träger hergestellt wird, worauf
b₁) im Falle von Kolonien mit zu testenden Nitrilhydratasegenen zuerst eine Inkubation der an dem Träger anhaftenden Zellen mit einer Substratlösung aus einem Nitril der Formel (l) R-CN, in der R einen gegebenenfalls ein- oder mehrfach mit unter den Reaktionsbedingungen inerten Substituenten substituierten C₁-C₂₀-Alkyl-, C₆-C₂₀-Aryl- oder C₅-C₂₀-Heteroarylrest bedeutet, und einem Puffer und anschließend
b₂) eine Inkubation mit einer gepufferten Hydroxylammoniumsalzlösung erfolgt, oder
c) im Falle von Zellkolonien mit zu testenden Amidasegenen eine Inkubation der an dem Träger anhaftenden Kolonien mit einer Substratlösung aus einem Amid der Formel (II) R-CONH₂, in der R wie oben definiert ist, Hydroxylammoniumsalz und einem Puffer erfolgt, worauf
d) im Anschluss an b₂) oder c) die aktiven Kolonien durch Überführen des Trägers in eine Eisen(III)salzlösung gefärbt werden und
e) gegebenenfalls Zellen von den aktiven Kolonien von dem Träger oder von den ursprünglichen Bereichen, in denen sich die Kolonien auf dem verfestigten Medium befunden haben, isoliert werden.

Mit der vorliegenden erfinderischen Screeningmethode kann Amidaseaktivität und indirekt Nitrilhydrataseaktivität auf einfache und kostengünstige Weise nachgewiesen werden.
Das Prinzip der erfindungsgemäßen Screeningmethode bzw. des verwendeten Screening assays beruht auf einer "Nebenaktivität" welche von einigen Amidasen, die in der Natur die Umwandlung von Amiden in Carbonsäuren katalysieren, gezeigt wird. Dies sind beispielsweise Amidasen von verschiedenen *Rhodococcus*-Stämmen, wie etwa *Rhodococcus sp.* R312, *Rhodococcus sp.* N774, *Rhodococcus rhodochrous* J1, u.s.w. oder von verschiedenen *Pseudomonas*-Stämmen, wie etwa *Pseudomonas aeruginosa, Pseudomonas chlororaphis* B23, u.s.w., die beispielsweise in Appl. Environ Microbiol, 1998 Aug, 64:8 2844-52 beschrieben sind. Sie katalysieren neben der Übertragung des Acylrestes eines Amids auf Wasser (=Hydrolyse des Amids) auch dessen Übertragung auf Hydroxylamin in der Gegenwart von Hydroxylammoniumsalzen unter der Bildung von Hydroxamsäuren. Hydroxamsäuren wiederum bilden mit Eisen(lll)-lonen tief rot bis violett gefärbte Komplexe, wodurch die Bildung der Hydroxamsäure und somit die Gegenwart der Amidaseaktivität (eigentlich "Transacylaseaktivität") colorimetrisch angezeigt wird, wie beispielsweise beschrieben in J Gen Microbiol. 1969, 57(2): 273-85 und Anal. Chem., 1952, 24(5), 898-900.

Nitrilhydratasen wandeln hingegen Nitrile in Amide um, die wie oben erwähnt, Substrate für Amidasen sind und somit entsprechend der erfindungsgemäßen Screeningmethode detektiert werden können.

Die Durchführung der erfindungsgemäßen Screeningmethode gliedert sich in 4 bzw. 5 Schritte.
Beim Nachweis von Amidaseaktivität wird im Schritt a) ein Replika von Zellkolonien, welche Amidasegene expremieren und die auf einem geeigneten Medium, wie etwa einer Kulturplatte bzw. einem verfestigten Medium, beispielsweise einer Agar-Platte, gewachsen sind, hergestellt. Die Herstellung des Repliks erfolgt durch Abnehmen bzw. "Abklatschen" von Zellmaterial der Kolonien auf der Platte mittels eines geeigneten Trägers, beispielsweise mittels einer Membran oder eines Filterpapiers. Dabei wird ein größerer Teil der in den Kolonien vorhandenen Zellen auf die Membran oder das Filterpapier übertragen wobei die räumliche Anordnung der Kolonien zueinander erhalten bleibt.
Als Membrane eignen sich dabei solche, die eine ausreichende Formstabilität und Haftfähigkeit von Zellen aus den Kolonien zeigen und eine geeignete Porosität aufweisen, wie etwa Polyamid-Membrane oder Nitrocellulose-Membrane. Wird eine hohe Auflösung gewünscht, so werden bevorzugt formstabile Nitrocellulose-Membrane eingesetzt, die beispielsweise die Herstellung präziser Replika von bis zu 5000 Kolonien auf einer Standard Agarplatte (Durchmesser etwa 9 cm) erlauben. Sind jedoch geringere Auflösungen von etwa 100 Kolonien ausreichend, so werden bevorzugt die wesentlich billigeren Filterpapiere verwendet.
Bei der Herstellung der Replika ist eine gute Haftung der Zellen am Trägermaterial von essentieller Bedeutung. Unterschiedliche Wirtsstämme, die bei der Klonierung verwendet werden, aber auch unterschiedliche Inkubationszeiten und Bedingungen können diese Eigenschaft maßgeblich beeinflussen.

Im nächsten Schritt, d.h. in Schritt c) erfolgt sodann die Inkubation der an der Membran oder dem Filterpapier anhaftenden Zellen mit der Substratlösung. Die Substratlösung besteht in diesem Fall aus einem Amid der Formel (II) R-CONH₂, einem Hydroxylammoniumsalz und einem Puffer.
In der Formel (II) bedeutet R einen gegebenenfalls ein- oder mehrfach mit unter den Reaktionsbedingungen inerten Substituenten substituierten C₁-C₂₀-Alkyl-, C₆-C₂₀-Aryl- oder C₅-C₂₀-Heteroarylrest.

Unter C₁-C₂₀-Alkyl sind dabei gesättigte oder ein- oder mehrfach ungesättigte, lineare, verzweigte oder cyclische, überbrückte, primäre, sekundäre oder tertiäre Hydrocarbonreste zu verstehen, wie etwa Methyl, Ethyl, Ethenyl, Propyl, i-Propyl, i-Propenyl, Butyl, i-Butyl, t-Butyl, Butenyl, Butinyl, Pentyl, Cyclopentyl, i-Pentyl, neoPentyl, Pentenyl, Hexyl, i-Hexyl, Cyclohexyl, Cyclohexylmethyl, 3-Methylpentyl, 2,2-Dimethylbutyl, 2,3-Dimethylbutyl, Octyl, Cyclooctyl, Decyl, Cyclodecyl, Dodecyl, Cyclododecyl u.s.w.
Bevorzugt sind dabei C₁-C₁₂-Alkylreste und besonders bevorzugt C₁-C₈-Alkylreste.

Die Alkylgruppe kann gegebenenfalls ein- oder mehrfach durch unter den Reaktionsbedingungen inerte Gruppen substituiert sein. Geeignete Substituenten sind beispielsweise gegebenenfalls substituierte Aryl- oder Heteroarylgruppen, wie Phenyl-, Naphthyl- oder Indolylgruppen, Halogen-, Hydroxy-, C₁-C₆-Alkoxy-, Aryloxy-, bevorzugt C₆-C₂₀-Aryloxy-, C₁-C₆-Alkylthio-, Cyano-, Amino-, Ether-, Thioether, Carbonsäureester-, Sulfoxid-, Sulfon-, Sulfonsäure, Sulfonsäureester-, Sulfinsäure-, Mercaptan-, Nitro- oder Azidogruppen.

Bevorzugte Substituenten sind dabei gegebenenfalls substituiertes Phenyl oder Naphthyl, Halogen, Hydroxy, Cyano, Amino, C₁-C₆-Alkoxy, C₁-C₆-Alkylthio- oder Phenoxy.

Unter Aryl sind bevorzugt C₆-C₂₀-Arylgruppen zu verstehen, wie etwa Phenyl, Biphenyl, Naphthyl, Indenyl, Fluorenyl u.s.w.

Die Arylgruppe kann dabei gegebenenfalls ein- oder mehrfach substituiert sein. Geeignete Substituenten sind dabei wiederum gegebenenfalls substituierte Aryl- oder Heteroarylgruppen, wie Phenyl-, Naphthyl- oder lndolylgruppen, Halogen-, Hydroxy-, C₁-C₆-Alkyl-, C₁-C₆-Alkoxy-, Aryloxy-, bevorzugt C₆-C₂₀-Aryloxy-, C₁-C₆-Alkylthio-, Cyano-, Amino-, Ether-, Thioether, Carbonsäureester-, Sulfoxid-, Sulfon-, Sulfonsäure, Sulfonsäureester-, Sulfinsäure-, Mercaptan-, Nitro- oder Azidogruppen.
Bevorzugte Substituenten sind dabei gegebenenfalls substituiertes Phenyl oder Naphthyl, Halogen, Hydroxy, Cyano, Amino, C₁-C₆-Alkoxy, C₁-C₆-Alkylthio- oder Phenoxy.

Unter Heteroaryl sind cyclische Reste zu verstehen, die mindestens ein S-, O- oder N-Atom im Ring enthalten. Dies sind beispielsweise Furyl, Pyridyl, Pyrimidyl, Thienyl, Isothiazolyl, Imidazolyl, Tetrazolyl, Pyrazinyl, Benzofuranyl, Benzothiophenyl, Chinolyl, lsochinolyl, Benzothienyl, Isobenzofuryl, Pyrazolyl, Indolyl, Isoindolyl, Benzoimidazolyl, Purinyl, Carbazolyl, Oxazolyl, Thiazolyl, Isothiazolyl, 1,2,4-Thiadiazolyl, Isoxazolyl, Pyrrolyl, Pyrrolidinyl, Chinazolinyl, Pyridazinyl, Phthalazinyl, Morpholinyl, u.s.w.
Funktionelle O- oder N-Gruppen können dabei nötigenfalls geschützt werden.
Die Heteroarylgruppe kann dabei gegebenenfalls ein- oder mehrfach durch die bereits oben angeführten Substituenten substituiert sein.

Geeignete Substrate sind demnach beispielsweise Acetamid, Propionamid, Butyramid, Isobutyramid, Valeramid, Pivalamide, Hexanoamid, Acrylamid, Methacrylsäureamid, Benzamid, Nicotinamid, lsonicotinamid, Lactamid, Alaninamid, Leucinamid, Methioninamid, Phenylalaninamid, Prolinamid, Threoninamid, Mandelsäureamid, 4-Cyano-3-Hydroxy-buttersäureamid u.s.w..

Als Hydroxylammoniumsalz eignen sich Chloride, Sulphate, sowie Salze aller anderen Anionen, die sich gegenüber der Reaktion neutral verhalten. Bevorzugt wird das handelsübliche Hydroxylammoniumchlorid eingesetzt.

Geeignete Puffer sind beispielsweise Phosphatpuffer, wie etwa Kaliumphosphat oder Natriumphosphat.

Die Inkubationslösung sollte dabei 0.5 -2 mol/l, bevorzugt 0.8 bis 1.5 mol/l Hydroxylammoniumsalz in 50 bis 200mM, bevorzugt 80 bis 150mM Puffer enthalten.

Das pH der gesamten Lösung muss an die Anforderung der spezifischen Amidase angepasst sein .
Bevorzugt wird bei einem pH-Wert im Bereich von pH 7 bis 8 gearbeitet.

Das Amid sollte in möglichst hohen Konzentrationen eingesetzt werden, wobei die Menge durch den Kostenfaktor und die Löslichkeit beeinflusst wird. Konzentration von ≥ 10mM sollten bevorzugt vorhanden sein, wobei Amide mit schlechten Substrateigenschaften bevorzugt in wesentlich größeren Konzentrationen von bis zu 1M zugesetzt werden.
Gegebenenfalls kann zur Erhöhung der Löslichkeit ein Cosolvens, bevorzugt aus der Gruppe DMSO, DMF oder C₁-C₄-Alkohol, in einer Menge von bis zu 20% der Endkonzentration zugegeben werden. Dieser Wert kann in Abhängigkeit von der Amidase auch noch überschritten werden.

Die Inkubationszeit kann dabei, in Abhängigkeit von der Reaktivität der Amide und der Enzymaktivität, von wenigen Minuten bis zu mehreren Stunden reichen, wobei kürzere lnkubationszeiten von 5 Minuten bis zu 30 Minuten bevorzugt sind.
Die Inkubationstemperatur wir in Abhängigkeit vom Optimum der eingesetzten Amidase gewählt und vorzugsweise bei ca. 20-40°C, meist um 30°C gehalten.

Zur Herstellung der Substratlösung wird dabei bevorzugt eine gepufferte Hydroxylammoniumsalz-Lösung mit einer Lösung des gewünschten Amids in Wasser oder einem Wasser/Cosolvens-Gemisch vermischt.

Bei Verwendung von Polyamid- oder Nitrozellulose- Membranen können diese zudem direkt mit den Kolonien nach oben auf einen Tropfen der Substratlösung gelegt werden. Filterpapiere benötigen größere Volumina und werden mit der Substratlösung getränkt.

Während der Inkubation bildet sich dabei in räumlicher Nähe zu aktiven Kolonien lokal begrenzt die entsprechende Hydroxamsäure.

Nach der Inkubation erfolgt gemäß Schritt d) das Färben der aktiven Kolonien. Dazu wird die Membran oder das Filterpapier in eine Eisen(lll)salzlösung, analog wie für die Substratlösung beschrieben, überführt.
Die Färbung erfolgt dabei unmittelbar bei Kontakt der Eisen(III)salz-Lösungmit der Hydroxamsäure.
Geeignete Eisen(lll)salze sind dabei übliche Salze, wie etwa Eisen(III)chlorid, Eisen(lll)sulphat, Eisen(III)nitrat u.s.w. Ein Nachteil von einfachen Eisen(lll)salzen ist jedoch ihre Hydrolyseempfindlichkeit bei neutralen und alkalischen pH-Werten, wodurch es zur Bildung von unlöslichen und stark gefärbten Hydrat- und Hydroxydniederschlägen kommen kann, die die Farbreaktion verschleiern oder verhindern können.
Um dies zu verhindern können stark saure Eisen(III)salz-Lösungen, beispielsweise Eisen(lll)chlorid in HCI, als Farbreagens eingesetzt werden. Der Nachteil dabei ist, dass die Bakterien- bzw. Zellkolonien im sauren Milieu absterben, weshalb die Zellen nur durch Lokalisierung und Abimpfen von der ursprünglichen Platte erhalten werden können.
Bevorzugt werden deshalb komplexe Eisensalze, wie etwa Ammoniumeisen(lll)citrat, Ammoniumeisen(III)sulphat u.s.w., in Wasser eingesetzt, die im neutralen Bereich noch nicht ausfallen.
Die Färbung erfolgt unmittelbar nach dem Kontakt mit dem Färbereagenz und hält meist für mehrere Minuten an (abhängig von der Menge der gebildeten Hydroxamsäure).
Bei der Verwendung von Membranen können diese wiederum direkt mit den Kolonien nach oben auf einen Tropfen der Färbelösung gelegt werden.
Filterpapiere enthalten eine entsprechend größere Menge an Substratlösung und müssen deshalb mit stark angesäuerten Eisen(lll)salzlösungen oder den komplexen Eisen(lll)salzlösungen gefärbt werden, um die Bildung der Hydroxydniederschläge zu vermeiden. Um die Menge an Substratlösung im Filtrierpapier zu verringern, kann dieses vor dem Färben auf trockenes Filterpapier gelegt werden. Alternativ dazu kann die Färbelösung direkt von oben auf die relevanten Bereiche aufgebracht werden.

Gemäß Schritt e) können Zellen von aktiven, nunmehr gefärbten Kolonien gegebenenfalls von der Membran oder dem Filterpapier isoliert und zur Weitervermehrung auf geeignete Nährmedien überimpft werden. Die Isolierung von Zellen ist auch von den ursprünglichen Bereichen auf der ursprünglichen Platte bzw. dem verfestigten Medium, auf denen sich die Kolonien befunden haben, möglich.

Bevorzugt werden die Zellen von aktiven Klonen jedoch von den übertragenen und gefärbten Kolonien auf den Membranen oder Filterpapieren durch direktes "Picken" isoliert.
Werden hohe Koloniedichten zum Screenen verwendet, so ist es von Vorteil die gepickten Klone über Vereinzelungsausstriche und erneutes Screenen zu reinigen.

Die Screeningmethode zum Nachweis von Amidaseaktivität ist in Figur 1 schematisch dargestellt. 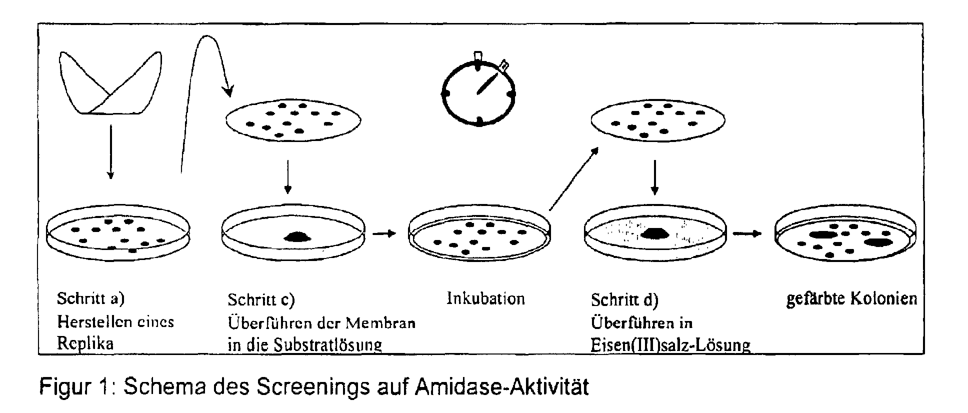 Nitrilhydratasen wandeln, wie oben beschrieben, Nitrile in Amide um. Da Amide Substrate für Amidasen sind, können diese mit der erfindungsgemäßen, oben beschriebenen Screeningmethode detektiert werden. Die Nitrilhydrataseaktivität wird erfindungsgemäß indirekt über das gebildete Amid mit Hilfe einer Amidase bestimmt.

Beim Nachweis von Nitrilhydrataseaktivität ist die variable Größe somit nicht mehr die Amidaseaktivität, sondern die Verfügbarkeit des Substrates, also des Amids R-CONH₂, das durch die Umwandlung eines Nitrils der Formel (I) R-CN, in der R wie oben definiert ist, mittels Nitrilhydratasen erhalten wird.

Um die Nitrilhydrataseaktivität nunmehr über das gebildete Amid zu ermitteln, ist es möglich, der Screeninglösung eine Amidase beizufügen.
Eine wesentlich günstigere Methode für das Identifizieren von Genen, die für Nitrilhydratase kodieren ist jedoch, einen Wirtsstamm zu verwenden, der eine geeignete Amidase expremiert. Wird DNA, die für Nitrilhydratase kodiert in einen solchen Wirtsstamm in geeigneter Weise eingebracht (beispielsweise über einen Plasmidvektor) so dass Expression des Nitrilhydratase Gens möglich ist, kann die Nitrilhydrataseaktivität bei Verwendung des erfindungsgemäßen Screeningsystems direkt erfasst werden. Es war daher auch Aufgabe dieser Erfindung, einen geeigneten Weg zur Entwicklung eines solchen Wirtsstammes zu finden. Als bevorzugte Lösung wurde gefunden, als Wirt rekombinante *E. coli* Stämme , die ein Amidasegen in das Chromosom integriert besitzen und von diesem aus Amidase expremieren bzw. das Enzym bilden, einzusetzen.
Die Herstellung solcher chromosomalen Integrationen kann nach unterschiedlichen Methoden erfolgen, wie zum Beispiel beschrieben in J. Bacteriol. 1997, **179**,(20): 6228-6237 (pKO3), J. Bacteriol. 1989, **171**(9): 4617-4622 (pSC101), J. Bacteriol. 2000, **182**(8): 2336-2340 (lambda-red) oder J. Bacteriol. 1984, **159**(2): 783-786 (lineare Fragmente).
In Zellen von solchen rekombinanten E.coli Stämmen mit Amidaseaktivität werden Vektoren, vorzugsweise Plasmide, welche Nitrilhydratase-Gene enthalten, eingeführt (z.B. durch Transformation), sodass in Kolonien von entstandenen rekombinanten Klonen enthaltene Nitrilhydratase-Aktivität mit Hilfe der vorher einebrachten und nunmehr "bakterieneigenen" Amidase nachgewiesen werden kann (wie in Figur 2 symbolisiert).

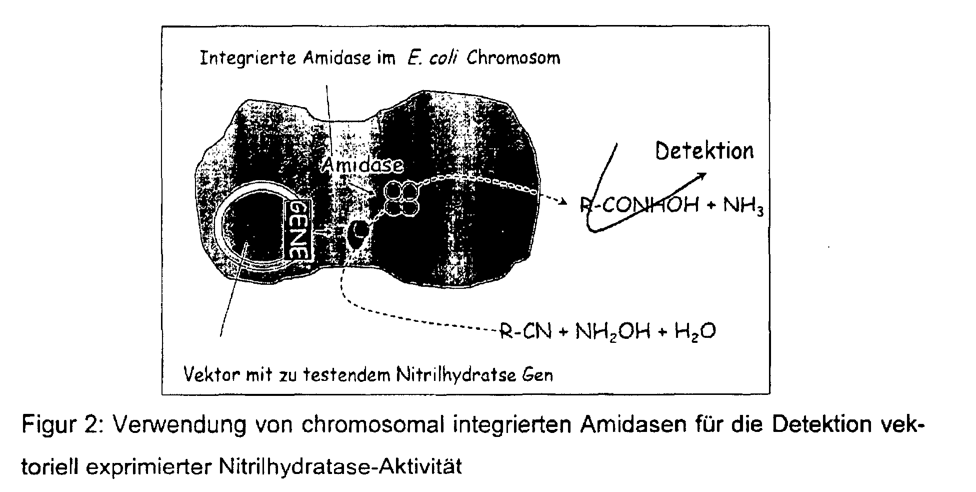

Neben solchen rekombinanten *E.coli* Stämmen können auch alle anderen Wirtsstämme verwendet werden, die eine starke Amidase (bzw. Acyltransferase)-Aktivität bei Abwesenheit einer Nitrilhydratase-Aktivität aufweisen.
Bei dieser Variante des erfindungsgemäßen Screeningverfahrens können Nitrilhydratasen durch Klonierung von DNA aus einem beliebigen Organismus (z.B nach Herstellung von Genbanken) unter Verwendung eines Wirtsstammes der eine geeignete Amidase expremiert nachgewiesen werden. Weiters ermöglicht dieses erfindungsgemäße Verfahren das Screenen nach verbesserten Nitrilhydtratase-Varianten, beispielsweise beim Einsatz von Strategien des "Directed Evolution"
Weiters kann eine Nitrilhydratase in solchen Stämmen (beispielsweise aus der Natur isolierte Stämme) gefunden werden, die sowohl Nitrilhydratase-Aktivität, wie auch Amidase-Aktivität aufweisen, wie dies etwa bei vielen Bakterien zu finden ist, häufig beispielsweise bei Vertretern der Gattung *Rhodococcus.*

Im Schritt a) wird dazu, analog der Vorgangsweise zum Nachweis von Amidaseaktivität, ein Replika von Zellkolonien mit zu testender Nitrilhydratase-Aktivität, die auf einer Kulturplatte bzw. einem verfestigten Medium, beispielsweise einer Agar-Platte, gewachsen sind, hergestellt.

Anschließend erfolgt im Schritt bᵢ) die Inkubation der an der Membran oder dem Filterpapier anhaftenden Zellen mit der Substratlösung. Die Substratlösung besteht in diesem Fall aus einem Nitril der Formel (I) R-CN, in der R wie oben definiert ist, und einem Puffer. Die Konzentration des Nitrils sollte wie zuvor bei den Amiden so hoch wie möglich liegen, ausgenommen jene Fälle, wo Substratinhibierung, starke Toxizität oder ein negativer Einfluss des Nitrits .auf die Amidase vorliegt, wobei Konzentrationen von 50 bis 200mM bevorzugt sind.

Als Puffer eignet sich dabei jeder Puffer, bei dem eine Nitrilhydratase-Aktivität erwartet werden kann, und der die vorhandene Amidase nicht irreversibel zerstört. Bevorzugt werden Phosphatpuffer mit einer Konzentration von 10-500mM verwendet.

Der pH-Wert kann von 2 bis 9 reichen, bevorzugt ist jedoch das pH-Optimum der Nitrilhydratase, das meist knapp bei pH 7 liegt.

Die Reaktionszeit richtet sich nach dem Typ des Substrates und der Nitrilhydratase und reicht von weniger als 1min bis mehr als 1h, bevorzugt im Bereich von 10-20min. Als Temperatur ist das Optimum des Enzyms, typisch zwischen Raumtemperatur und 40°C, häufig ca. 25-30°C zu verwenden.

Bei mangelnder Löslichkeit des Nitrils ist der Einsatz von Cosolventien, bevorzugt aus der Gruppe DMSO, DMF oder C₁-C₄-Alkohol möglich, vorausgesetzt die Nitrilhydratase-, wie auch die Amidase-Aktivität bleiben dabei erhalten.

Bei diesem Schritt bilden Nitrilhydratase-aktive Zellen das korrespondierende Amid der Formel (II) R-CONH₂.

Da Amidasen im allgemeinen langsamer arbeiten als Nitrilhydratasen, wird in dieser Zeit noch nicht das gesamte Amid zur Carbonsäure weiter hydrolysiert.

In Schritt b₂) erfolgt sodann die Inkubation mit einer gepufferten Hydroxylammoniumsalz-Lösung.
Als Hydroxylammoniumsalz eignen sich wiederum Chloride, Sulphate und alle gängigen Anionen, die nicht direkt in die Reaktion eingreifen. Bevorzugt wird das handelsübliche Hydroxylammoniumchlorid eingesetzt.
Geeignete Puffer sind beispielsweise Phosphatpuffer, wie etwa Kaliumphosphat oder Natriumphosphat wie auch die meisten anderen gebräuchlichen Puffer.
Die Inkubationslösung sollte dabei 0.5 bis 2mol/l, bevorzugt 0.8 bis 1.5mol/l Hydroxylammoniumsalz in 50 bis 200mM, bevorzugt 80 bis 150mM Puffer enthalten.
Der pH der gesamten Lösung muss an die Anforderung der integrierten Amidase angepasst sein und liegt bevorzugt um den Bereich von pH 7 bis 8. Auch die Inkubationszeit richtet sich nach den verwendeten Substrat/Enzym-Kombinationen. Typischer Weise werden Inkubationszeiten von 10-30min bevorzugt, um eine zu weit fortschreitende Diffusion zu verhindern. Cosolventien werden für diese Stufe des Screenings meist nicht benötigt, da die Löslichkeit des sich bildenden Amids meist weit über der des zugrundeliegenden Nitrils liegt.

Die Amidase überträgt nun von dem gebildeten Amid die Acylreste auf das Hydroxylamin und erzeugt so eine Hydroxamsäure, die in Schritt d) wie oben beschrieben einer mit Eisen(lll)salzlösung gefärbt wird.
Schritt e) erfolgt ebenfalls wie oben beschrieben.

In Figur 3 ist die Screeningmethode zum Nachweis von Nitrilhydratase-Aktivität dargestellt: 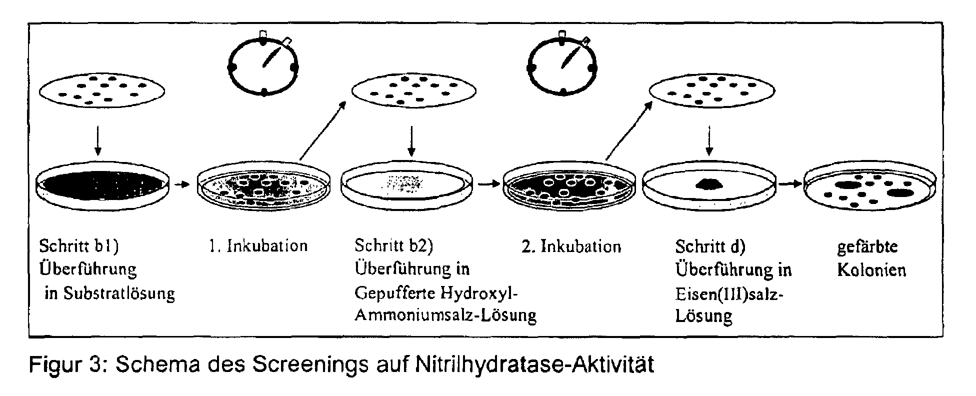 Die erfindungsgemäße Screeningmethode auf Kolonie-Ebene ("Colony Assay") kann vielfältig zum Nachweis von Amidaseaktivität bzw. Nitrilhydrataseaktivität eingesetzt werden.
So kann die Methode beispielsweise zum Nachweis von Amidaseaktivität in genomischen oder cDNA Genbanken eingesetzt werden. Als Ursprungsorganismus eignen sich dabei grundsätzlich alle Organismen, beispielsweise Bakterien aus der Gattung *Rhodococcus,* wie etwa *Rhodococcus equi oder Rhodococcus ruber,* den Gattungen *Pseudomonas oder Acinetobacter,* sowie allen anderen, die Gene des gesuchten Typs enthalten. Voraussetzung ist, dass diese Gene in den verwendeten Wirtstämmen expremiert werden.
Weiters kann die erfindungsgemäße Screeningmethode zum Nachweis von erhöhter Amidaseaktivität und von erhöhter Nitrilhydratase-Aktivität und Stabilität in Genbanken, die für Mutanten oder Rekombinanten solcher Enzyme codierende Gene enthalten (wie beispielsweise im Verlauf von Strategien des "Directed Evolution" hergestellt), verwendet werden. Eine weitere Anwendungsmöglichkeit ist bei standardmäßigen Klonierungsarbeiten von Amidase- oder Nitrilhydratase-Genen wie beispielsweise die schnelle Erkennung und Isolierungsmöglichkeit von Amidase- oder Nitrilhydratase-aktiven Klonen bei laborüblichen Umklonierungsarbeiten, etwa aus einem Transformationsgemisch mit einer großen Anzahl von inaktiven Zellen, wodurch viel Zeit und Material gespart werden kann. Beispielsweise kann die erfindungsgemäße Screeningmethode zur Isolation von Nitrilhydratasegenen aus Genbanken die in erfindungsgemäßen rekombinanten E. *coli* Wirtsstämmen unter Verwendung geeigneter Vektoren, beispielsweise von Plasmiden verwendet werden.

Weitere Vorteile der erfindungsgemäßen Screeningmethode bzw. des "Colony Assay" sind die Möglichkeit hoher Durchsätze und der geringe Kostenfaktor, sowie die Anwendbarkeit auf heterolog exprimierende Klone wie auch, mit beschriebenen Einschränkungen für das Nitrilhydratase-screening, auf "Wild Type" Organismen.

Die erfindungsgemäße Screeningmethode kann erfindungsgemäß zum Klonieren eines Amidase- oder eines Nitrilhydratasegens verwendet werden.

Viele in der Literatur beschriebene Amidasen und Nitrilhydratasen wurden aus Bakterien der Gattung *Rhodococcus, Pseudomonas oder Acinetobacter* isoliert. Diese zeigen vielfach nützliche Eigenschaften für die Biokatalyse, sodass die Nachfrage nach Amidaseklonen oder Nitrilhydrataseklonen auch weiterhin steigt.

Eine weitere Aufgabe der vorliegenden Erfindung war es demnach eine Klonierungsmöglichkeit für Amidasen und Nitrilhydratasen zu finden, die ohne die Einschränkungen der Homologiemethoden (PCR, Hybridisierungstechniken) oder den aufwendigen Weg der Proteinreinigung und -sequenzierung funktioniert.
Diese Aufgabe wurde mit der erfindungsgemäßen Screeningmethode, bzw. mit dem erfindungsgemäßen Kolonie Assay gelöst.

Ein weiterer Gegenstand der vorliegenden Erfindung ist demnach die Verwendung der oben beschriebenen Screeningmethode zum Nachweis von Amidase-Aktivität bei der Klonierung eines Amidasegens oder eines Nitrilhydratasegens aus Organismen, vorzugsweise aus Bakterien wie z.B. *Rhodococcus, Pseudomonas oder Acinetobacter* Bakterien.

Dazu wird zuerst eine chromosomale Genbank des entsprechenden Stammes, beispielsweise eines Rhodococcus Stammes, gemäß dem Stand der Technik erstellt. Dabei wird beispielsweise *Rhodococcus equi* auf einem geeigneten Vollmedium, angezüchtet und (A) die chromosomale DNA nach einer Standardmethode isoliert. Geeignet sind alle Methoden die saubere DNA-Fraktionen hohen Molekulargewichts liefern, eingeschlossen der kommerziell erhältlichen DNA-lsolation-Kits.
Anschließend wird (B) die chromosomale DNA mit einem Restriktionsenzym partiell so verdaut, dass Fragmente mit einer geeigneten Größe, im Fall von Bakterien vorzugsweise von etwa 2-10 kb erhalten werden. Diese Fragmente werden dann, gegebenenfalls nach Reinigung, beispielsweise über präparative Gelelektrophorese, oder mittels Saccharose- Dichtegradientenzentrifugation, (C) in geeignete Vektoren, vorzugsweise Plasmide wie beispielsweise in mit BamHl-linearisierte, dephosphorylierte pBluescript SK― Plasmide, einkloniert. Als Plasmid eignet sich dabei prinzipiell jedes autonom replizierende Plasmidkonstrukt, welches eine Expression enthaltener Gene von Plasmid- oder geneigenen Promotoren aus erlaubt. Anschließend erfolgt (D) der Einbau (beispielsweise durch Transformation) in geeignete Wirtszellen, wie etwa kompetente *Escherichia coli* Zellen, wodurch eine Genbank mit zehntausenden Klonen erhalten wird, welche konserviert werden.

Für das Klonieren von Nitrilhydratasen wird bei (D) eine Transformation in geeignete Wirtszellen, wie etwa kompetente *E.coli* Zellen vorgenommen, welche eine chromosomale Integration einer Amidase, beispielsweise einer Amidase einer *Rhodococcus* Spezies, besitzen.

Als nächster Schritt erfolgt das Screenen der Genbank nach Amidase- bzw. Nitrilhydratasen-aktiven Klonen.
Dazu werden bevorzugt Verdünnungen der Genbank auf geeigneten verfestigten Medien, beispielsweise Agar-Platten ausplattiert und einige Zeit inkubiert, wodurch Platten mit Kolonien, vorzugsweise im Bereich von 1000 - 3000 Kolonien pro Platte, erhalten werden.

Von diesen Kolonien wird gemäß dem erfindungsgemäßen Screeningsystem in Schritt a) ein Replika hergestellt, das gemäß Schritt b₁) und b₂) (Nitrilhydratasen) oder gemäß Schritt c) (Amidasen) mit der Substratlösung inkubiert wird und anschließend gemäß Schritt d) gefärbt wird. Anschließend werden gemäß Schritt e) positive bzw. aktive Klone isoliert, beispielsweise durch Abnehmen mit sterilen Zahnstochern. Durch die hohe Koloniendichte ist es von Vorteil, Vereinzelungsausstriche zu kultivieren und die Screeningmethode erneut anzuwenden, wodurch Reinkulturen der positiven bzw. aktiven Amidase- oder Nitrilhydratase-Klone erhalten werden.
Zum Abschluss können die Klone mittels Restriktionsanalyse auf ihre Insertgröße getestet werden und bei Bedarf der für die Enzymfunktion notwendige DNA Abschnitt durch Subklonierung auf eine minimale Größe reduziert werden, wobei das erfindungsgemäße Screeningverfahren wiederum zu Selektion von Klonen die aktives Enzym expremieren herangezogen werden kann.

Mit Hilfe der erfindungsgemäßen Screeningmethode bzw. des erfindungsgemäßen "Colony Assays" kann somit ein für eine Amidase oder eine Nitrilhydratase codierendes Gen gänzlich ohne die Zuhilfenahme von Sequenzinformation (etwa abgeleitet aus Homologien oder Peptidsequenzierung) mit geringem zeitlichen Aufwand kloniert werden. Auch bei der Unterstützung von einfachen Klonierarbeiten erweist sich der Assay als sehr nützlich bei der Identifizierung von positiven Klonen.

Der wie oben beschriebene Genbankklon kann sodann sequenziert werden und der Leserahmen mit geeigneten Primern mittels PCR amplifiziert und über primerspezifische Schnittstellen in einen geeigneten Überexpressionsvektor einkloniert werden, wodurch Plasmide erhalten werden, die eine starke Überexpression des Enzyms in einem geeigneten Organismus, wie etwa *E. coli* erlauben.

Weiters erlaubt die erfindungsgemäße Screeningmethode Abstufungen verschiedener Aktivitätsniveaus von Amidasen und Nitrilhydratasen in Zellkolonien zu detektieren. Intensivere Färbung korreliert dabei mit einem höheren Aktivitätsniveau. Weiters können durch Variation der Inkubationszeiten während den Schritten b₁ bzw. c die Detektionsfenster an die gegebenen Aktivitäten angepasst werden. Diese Vorgehensweise erlaubt eine Nutzung der Screeningmethode als Ausleseverfahren für beispielsweise "directed evolution"-Experimente oder andere Mutagenese- oder Rekombinations-Experimente zur Gewinnung von verbesserten Varianten von Amidasen und Nitrilhydratasen durch die Unterscheidung von aktiveren von weniger aktiven Mutanten. Die erfindungsgemäße Methode kann damit zum Beispiel für die Isolation von aktiveren oder stabileren Klonen aus für Enzymvarianten codierenden Genbibliotheken (z.B. "random Mutagenese" Bibliotheken, "gene shuffling" Bibliotheken) genutzt werden.

### Beispiel 1:

### Erstellung einer chromosomalen Genbank von Rhodococcus equi IFO3730

Rhodococcus *equi* wurde auf Luria Broth (LB)-Agar-Medium angezüchtet und die chromosomale DNA nach einer Standardmethode wie in Ausubel et al., Current Protocols, Wiley and Sons, 2002) beschrieben gewonnen (Lysozym/SDS/CTAB-Aufschluß, CIP-Extraction, Isopropanolfällung). Die chromosomale DNA wurde anschließend mit dem Restriktionsenzym Sau3Al partiell verdaut, sodass Fragmente der Größe 2-10kb erhalten wurden. Diese Fragmente wurden anschließend über präparative Gelelektrophorese gereinigt und in mit BamHl-linearisierte, dephosphorylierte pBluescript SK―Plasmide ligiert. Die Transformation in elektrokompetente E*scherichia coli* TOP10F' Zellen durch Elektroporation resultierte in einer Genbank mit 70000 Klonen welche konserviert wurden.

### Screenen der Genbank nach Amidase-aktiven Klonen

Es wurden Verdünnungen der Genbank auf LB- Agar-Platten, supplementiert mit 100 *mg*/*L* Ampicillin (LB-Amp-Platten) ausplattiert und über Nacht inkubiert, wodurch ca. 2000 Kolonien pro Platte erhalten wurden. Zellen von diesen Kolonien wurden mit Nitrocellulosemembranen (NC-extra 0.2µm Sartorius) abgenommen und in der Substratlösung (1M Hydroxylammoniumchlorid, 100mM Kaliumphosphat, 2.5% Acetamid; pH=7.5) für 20min bei 37°C inkubiert. Anschließend wurden die inkubierten Membranen mit einer 1M Ammoneisen(III)citratlösung gefärbt und positive Klone von der Membran mittels steriler Zahnstocher abgenommen.
Die über Nacht kultivierten Vereinzelungsausstriche wurden erneut getestet um schließlich Reinkuluren der positiven Klone zu erhalten. Die Klone wurden im Anschluss mittels Restriktionsanalyse (Schneiden mit *EcoR*I und *Xbal)* auf ihre Insertgröße getestet. pBS_IFO_amd_3 wies dabei ein relativ kleines Insert von nur 2.5kb auf. Von diesem Klon wurde die Plasmid DNA isoliert und für eine Sequenzierung des Inserts herangezogen.

### Umklonierung des Amidase-Gens vom pBluescript-Vektor in den Überexpressionsvektor pMS470Δ8

Die Sequenzierung des Genbankklons lieferte schließlich einen offenen Leserahmen welcher Homologie zu bekannten Amidasen *(Brevibacterium sp.* R312) aufwies. Dieser Leserahmen wurde mit Primern (rh_equi_IFO_amd3_f1 und _r1) mittels PCR (Pwo-Polymerase = proofreading Enzym) amplifiziert und über die primerspezifischen Schnittstellen (Ndel und *Sphl)* in den vorbereiteten pMS470Δ8 Vektor (**Balzer, D., G. Ziegelin, W. Pansegrau, V. Kruft, and E. Lanka.** 1992. KorB protein of promiscuous plasmid RP4 recognizes inverted sequence repetitions in regions essential for conjugative plasmid transfer. Nucl. Acids Res. **20**: 1851-1858.), nach Schneiden mit den gleichen Enzymen und Gewinnung des großen Vektor-Fragments, einkloniert. Das so erhaltene Plasmid (pMS470-48_2_1) wurde in *Escherichia coli BL21* transformiert und erlaubte eine starke Überexpression des Enzyms in E. *coli,* induzierbar durch IPTG oder Lactose .

## Patentansprüche

1. Screeningmethode zum Nachweis von Amidase- oder Nitrilhydrataseaktivität, **dadurch gekennzeichnet, dass**
a) ein Replika von Zellkolonien, die Amidasen oder Nitrilhydratasen expremieren und die auf einem geeigneten, verfestigtem Medium gewachsen sind, auf einem geeigneten Träger hergestellt wird, worauf
b₁) im Falle von Kolonien mit zu testenden Nitrilhydratasegenen zuerst eine lnkubation der an dem Träger anhaftenden Zellen mit einer Substratlösung aus einem Nitril der Formel (l) R-CN, in der R einen gegebenenfalls ein- oder mehrfach mit unter den Reaktionsbedingungen inerten Substituenten substituierten C₁-C₂₀-Alkyl-, C₆-C₂₀-Aryl- oder C₅-C₂₀-Heteroarylrest bedeutet, und einem Puffer und anschließend
b₂) eine Inkubation mit einer gepufferten Hydroxylammoniumsalzlösung erfolgt, oder
c) im Falle von Zellkolonien mit zu testenden Amidasegenen eine Inkubation der an dem Träger anhaftenden Kolonien mit einer Substratlösung aus einem Amid der Formel (II) R-CONH₂, in der R wie oben definiert ist, Hydroxylammoniumsalz und einem Puffer erfolgt, worauf
d) im Anschluss an b₂) oder c) die aktiven Kolonien durch Überführen des Trägers in eine Eisen(III)salzlösung gefärbt werden und
e) gegebenenfalls Zellen von den aktiven Kolonien von dem Träger oder von den ursprünglichen Bereichen, in denen sich die Kolonien auf dem verfestigtem Medium befunden haben, isoliert werden.

2. Screeningmethode nach Anspruch 1, **dadurch gekennzeichnet, dass** als Träger Membrane oder Filterpapier verwendet werden.

3. Screeningmethode nach Anspruch 1, **dadurch gekennzeichnet, dass** für die Substratlösungen in Schritt b₁) Nitrile der Formel (l) R-CN und in Schritt c) Amide der Formel (II) R-CONH₂ eingesetzt werden, worin R einen gesättigten oder einfach ungesättigten, linearen, verzweigten oder cyclischen C₁-C₁₂-Alkylrest oder einen Phenyl-, Biphenyl- oder Naphthylrest oder einen Pyrrolidinylrest bedeutet, der gegebenenfalls ein- oder mehrfach durch Substituenten aus der Gruppe Halogen, Hydroxy, Cyano, Amino, gegebenenfalls substituiertes Phenyl oder Naphthyl, C₁-C₆-Alkyl, C₁-C₆-Alkoxy, C₁-C₆-Alkylthio oder Phenoxy substituiert sein kann.

4. Screeningmethode nach Anspruch 1, **dadurch gekennzeichnet, dass** als Hydroxylammoniumsalz ein Chlorid oder ein Sulfat eingesetzt wird.

5. Screeningmethode nach Anspruch 1, **dadurch gekennzeichnet, dass** als Puffer Phosphatpuffer eingesetzt werden.

6. Screeningmethode nach Anspruch 1, **dadurch gekennzeichnet, dass** die Inkubationslösungen in Schritt b₂) und c) 0.5 -2mol/l Hydroxylammoniumsalz in 50 bis 200mM Puffer enthalten.

7. Screeningmethode nach Anspruch 1, **dadurch gekennzeichnet, dass** in Schritt b₁) und in Schritt c) dem Substrat gegebenenfalls ein Cosolvens aus der Gruppe DMSO, DMF oder C₁-C₄-Alkohol zugesetzt wird.

8. Screeningmethode nach Anspruch 1, **dadurch gekennzeichnet, dass** in Schritt d) als Eisen(III)salzlösung stark angesäuerte Eisen(lll)chlorid-, Eisen(lll)sulphat- oder Eisen(III)nitratlösungen oder Lösungen von komplexen Eisen(lll)salzen aus der Gruppe Ammoniumeisen(III)citrat oder Ammoniumeisen(lll)sulphat eingesetzt werden.

9. Screeningmethode nach Anspruch 1, **dadurch gekennzeichnet, dass** beim Nachweis von Nitrilhydrataseaktivität der Screeninglösung in Schritt b₁) eine Amidase zugesetzt wird.

10. Screeningmethode nach Anspruch 1, **dadurch gekennzeichnet, dass** eine in einen E. *coli* Stamm chromosomal integrierte Amidase für den Nachweis vektoriell expremierter Nitrilhydrataseaktivität eingesetzt wird.

11. Verwendung der Screeningmethode gemäß Anspruch 1 zum Nachweis von Amidase-Aktivität bei der Klonierung eines Amidasegens aus geeigneten Organismen.

12. Verwendung der Screeningmethode gemäß Anspruch 1 zum Nachweis von Amidase-Aktivität bei der Klonierung eines Amidasegens aus Bakterien.

13. Verwendung der Screeningmethode nach Anspruch 12, **dadurch gekennzeichnet** das als Bakterien *Rhodococcus-, Pseudomonas- oder* Acinetobacter-Bakterien verwendet werden.

14. Verwendung der Screeningmethode gemäß Anspruch 1 zum Nachweis von Nitrilhydratase-Aktivität bei der Klonierung eines Nitrilhydratasegens aus geeigneten Organismen.

15. Verwendung der Screeningmethode gemäß Anspruch 1 zum Nachweis von Nitrilhydratase-Aktivität bei der Klonierung eines Nitrilhydratasegens aus Bakterien.

16. Verwendung der Screeningmethode nach Anspruch 15, **dadurch gekennzeichnet** das als Bakterien *Rhodococcus-, Pseudomonas- oder Acinetobacter* -Bakterien verwendet werden.

17. Verwendung der Screeningmethode gemäß Anspruch 1 zum Nachweis von erhöhter Amidase-Aktivität in random Mutagenese-Bibliotheken.

18. Verwendung der Screeningmethode gemäß Anspruch 1 zum Nachweis erhöhter Nitrilhydratase-Aktivität und Stabilität in random Mutagenese-Bibliotheken.

19. Verwendung der Screeningmethode gemäß Anspruch 1 zur Isolation von Nitrilhydratasegenen aus Genbanken in *E.* coli-Plasmiden.
